(19) Europäisches Patentamt
European Patent Office
Office européen des brevets

(11) Publication number : **0 479 481 A2**

# (12) EUROPEAN PATENT APPLICATION

(21) Application number : **91308722.7**

(51) Int. Cl.[5] : **C07K 5/10,** A61K 37/64

(22) Date of filing : **25.09.91**

(30) Priority : **27.09.90 US 589282**

(43) Date of publication of application : **08.04.92 Bulletin 92/15**

(84) Designated Contracting States : **AT BE CH DE DK ES FR GB GR IT LI LU NL SE**

(71) Applicant : **MERCK & CO. INC.
126, East Lincoln Avenue P.O. Box 2000
Rahway New Jersey 07065-0900 (US)**

(72) Inventor : **Duggan, Mark E.
300 North Essex Avenue, Apt. 205B
Narberth, PA 19072 (US)**

(74) Representative : **Barrett-Major, Julie Diane et al
Merck & Co., Inc. European Patent Department Terlings Park Eastwick Road
Harlow Essex CM20 2QR (GB)**

## (54) Fibrinogen receptor antagonists.

(57) Fibrinogen receptors antagonists of the Formula :

$$(X-(CH_2)_m-Y-(CH_2)_n-\overset{O}{\overset{\|}{C}})\text{-Gly-Asp-AA} \qquad \text{I}$$

are disclosed for use in inhibiting the binding of fibrinogen to blood platelets and inhibiting the aggregation of blood platelets.

EP 0 479 481 A2

## FIELD OF THE INVENTION

This invention relates to the discovery of fibrinogen receptor antagonists of Formula I for use in inhibiting the binding of fibrinogen to blood platelets and inhibiting the aggregation of blood platelets when administered to mammals, preferably humans.

## BACKGROUND OF THE INVENTION

The interaction of platelets with the coagulation and fibrinolytic systems in the maintenance of hemostasis may become pathogenic, requiring prevention and treatment. The fibrinogen receptor antagonists of Formula I are useful in treating various diseases related to platelet aggregation and fibrin formation.

A renewed interest in platelet inhibitors has reemerged as a result of a better understanding of the role of platelets and thrombosis in the pathogenesis of vascular disease, including unstable angina, acute myocardial infarction and stroke.

Platelets are cell-like anucleated fragments, found in the blood of all mammals, which participate in blood coagulation. Fibrinogen is a glycoprotein present as a normal component of blood plasma. Fibrinogen participates in platelet aggregation and fibrin formation in the blood clotting mechanism. Platelets are deposited at sites of vascular injury where multiple physiological agonists act to initiate platelet aggregation culminating in the formation of a platelet plug to minimize blood loss. If the platelet plug occurs in the lumen of a blood vessel, normal blood flow is impaired.

Platelet membrane receptors are essential in the process of platelet adhesion and aggregation. Interaction of fibrinogen with a receptor on the platelet membrane complex IIb/IIIa is known to be essential for normal platelet function.

Zimmerman et al., U.S. Patent No. 4,683,291, describes peptides having utility in the study of fibrinogen-platelet, platelet-platelet, and cell-cell interactions. The peptides are described as having utility where it is desirable to retard or prevent formation of a thrombus or clot in the blood. The general formula for the peptides is:

$H_2N$-(Ch)-Arg-Gly-Asp-(Cx)-H

where Ch and Cx are sequences of amino acids.

Pierschbacher et al., U.S. Patent No. 4,589,881, describes the sequence of an 11.5 kDal polypeptide fragment of fibronectin which embodies the cell-attachment-promoting activity of fibronectin. A specifically described fragment is:

H-Tyr-Ala-Val-Thr-Gly-Arg-Gly-Asp-
Ser-Pro-Ala-Ser-Ser-Lys-Pro-Ile-
Ser-Ile-Asn-Tyr-Arg-Thr-Glu-Ile-
Asp-Lys-Pro-Ser-Gln-Met-OH

Ruoslahti et al., U.S. Patent No. 4,614.517, describes tetrapeptides which alter cell-attachment activity of cells to various substrates. The peptides are stated to "consist essentially of" the following sequence:

X-Arg-Gly-Asp-Ser-Y

wherein X is H or one or more amino acids and Y is OH or one or more amino acids. Figure 1 lists the polypeptides that were synthesized by Ruoslahti et al. in "determining the smallest peptide exhibiting cell attachment activity". Ruoslahti et al., U.S. Patent No. 4,578,079, describes similar tetrapeptides having Ser substituted with Thr or Cys.

Pierschbacher et al., Proc. Natl. Acad. Sci USA, Vol. 81, pp.5985-5988, October, 1984, describe variants of the cell recognition site of fibronectin that retain attachment-promoting activity. Pierschbacher et al., further assayed the cell attachment-promoting activities of a number of structures closely resembling the Arg-Gly-Asp-Ser peptide, and found "that the arginine, glycine, and aspartate residues cannot be replaced even with closely related amino acids, but that several amino acids can replace serine without loss of activity."

Ruoslahti et al., Science, Vol. 238, pp. 491-497, October 23, 1987, discuss cell adhesion proteins. They specifically state that "[e]lucidation of the amino acid sequence of the cell-attachment domain in fibronectin and its duplication with synthetic peptides establish the sequence Arg-Gly-Asp (RGD) as the essential structure recognized by cells in fibronectin".

Cheresh, Proc. Natl. Acad. Sci. USA, Vol. 84, pp. 6471-6475, September 1987, describes the Arg-Gly-Asp-directed adhesion receptor involved in attachment to fibrinogen and the von Willebrand Factor.

Adams et al., U. S. Patent No. 4,857,508, describes tetrapeptides which inhibit platelet aggregation and

the formation of a thrombus. The tetrapeptides have the formula:

X-Gly-Asp-Y

wherein X can be H2NC(=NH)NH(CH2)nCH(Z)COOH or Ac-Arg, wherein Z = H, NH2, or NH-Acyl and n=1-4, and wherein Y can be Tyr-NH2, Phe-NH2 or a group of a specifically defined formula.

It is, therefore, an object of the present invention to provide fibrinogen receptor antagonists for use in inhibiting the binding of fibrinogen to blood platelets and inhibiting the aggregation of blood platelets. Another aspect of the present invention is to provide novel fibrinogen receptor antagonist compounds. Other objects of the present invention are to provide methods of inhibiting the binding of fibrinogen to blood platelets and inhibiting the aggregation of blood platelets through the administration of novel fibrinogen receptor antagonist compounds. The above and other objects are accomplished by the present invention in the manner more fully described below.

## SUMMARY OF THE INVENTION

The present invention provides fibrinogen receptor antagonist compounds of the Formula:

$$(X-(CH_2)_m-Y-(CH_2)_n-\overset{O}{\overset{\|}{C}})-Gly-Asp-AA \qquad \text{I}$$

for use in inhibiting the binding of fibrinogen to blood platelets and inhibiting the aggregation of blood platelets. The above-mentioned compounds can be used in a method of acting upon a fibrinogen receptor which comprises administering a therapeutically effective but non-toxic amount of such compound to a mammal, preferably a human. A pharmaceutical composition comprising a pharmaceutically acceptable carrier and, dispersed therein, an effective but non-toxic amount of such compound is another feature of this invention.

## DETAILED DESCRIPTION OF THE INVENTION

Fibrinogen receptor antagonist compounds of Formula I are useful in a method of inhibiting the binding of fibrinogen to blood platelets and for inhibiting the aggregation of blood platelets. Fibrinogen receptor antagonists of this invention are illustrated by compounds having the formula:

$$(X-(CH_2)_m-Y-(CH_2)_n-\overset{O}{\overset{\|}{C}})-Gly-Asp-AA$$

wherein:

X is

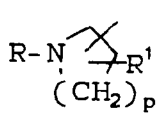 , 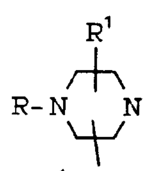 ,

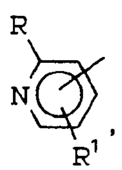 , 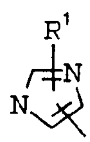 ,

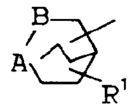 or 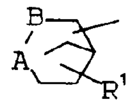 ;

A or B is N-R, provided that when A is N-R, B is -CH2-, and that when B is N-R, A is

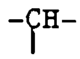 ;

Y is

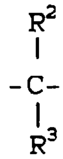 , 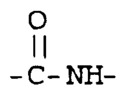 , O,

N-$R^2$ , 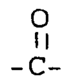 , $-S(O)_q-CH_2-$,

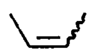 , 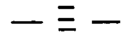 ,

$-SO_2NH-$, or 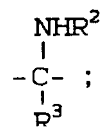 ;

R, $R^1$, $R^2$ and $R^3$ are independently alkyl, aryl, alkylaryl, substituted alkyl, or H;
AA is an L-Amino Acid;
m is 1-10;
n is 0-9;
q is 0-2;
p is 1-6;
and the pharmaceutically acceptable salts thereof.

In a preferred embodiment of the present invention, the fibrinogen receptor antagonist has the following formula:

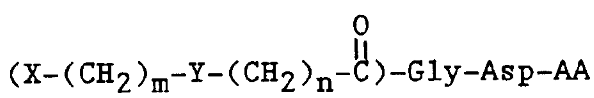

I

wherein:

X is

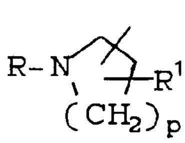 or 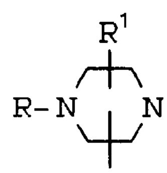 ;

Y is

$$-\overset{R^2}{\underset{R^3}{C}}-,\ O,\ or\ -S(O)_q-CH_2-;$$

R, $R^1$, $R^2$ and $R^3$ are independently alkyl, aryl, alkylaryl, substituted alkyl, or H;
AA is an L-Amino Acid;
m is 1-5;
n is 0-5;
q is 0-2;
p is 2-4;
and the pharmaceutically acceptable salts thereof.

A preferred compound of this invention is set forth below:

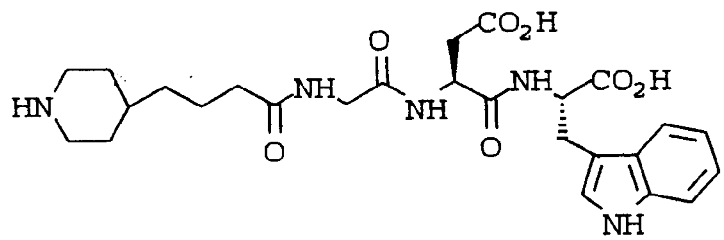

This compound may alternatively be named Pib-Gly-Asp-Trp.

The Amino Acids (AA) that can be utilized in this invention are L-Amino Acids and are set forth as follows: Ala, Alanine; Arg, Arginine; Asn, Asparagine; Asp, Aspartic Acid; Cys, Cysteine; Gln, Glutamine; Glu, Glutamic Acid; Gly, Glycine; His, Histidine; Ile, Isoleucine; Leu, Leucine; Lys, Lysine; Met, Methionine; Phe, Phenylalanine; Pro, Proline; Ser, Serine; Thr, threonine; Trp, Tryptophan; Tyr, Tyrosine; and Val, Valine. These standard abbreviations are well recognized in the art of peptide chemistry. Asp in Formula I of this invention is an L-Amino Acid.

In addition to the three letter abbreviations used to identify amino acids, this invention includes the following abbreviation designations; Bn, benzyl; NMM, N-methylmorphline; HOBt, 1-hydroxybenzotriazole; EDC, 1-(3-dimethylaminopropyl)-3-ethyl-carbodiimide-hydrochloride; DMF, dimethylformamide; Pib, 4-(4 piperidyl)butanoyl; and Boc-Asp(Bn), N-Boc-Asp-β-benzyl ester.

The pharmaceutically acceptable salts of the compounds of Formula I include the conventional non-toxic salts or the quarternary ammonium salts of the compounds of Formula I formed, e.g., from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

The pharmaceutically acceptable salts of the present invention can be synthesized from the compounds of Formula I which contain a basic or acidic moiety by conventional chemical methods. Generally, the salts are prepared by reacting the free base or acid with stoichiometric amounts or with an excess of the desired salt-forming inorganic or organic acid or base in a suitable solvent or various combinations of solvents.

The pharmaceutically acceptable salts of the acids of Formula I are also readily prepared by conventional procedures such as treating an acid of Formula I with an appropriate amount of a base, such as an alkali or alkaline earth metal hydroxide e.g. sodium, potassium, lithium, calcium, or magnesium, or an organic base such as an amine, e.g., dibenzylethylenediamine, trimethylamine, piperidine, pyrrolidine, benzylamine and the like, or a quaternary ammonium hydroxide such as tetramethylammonium hydroxide and the like.

The compounds of Formula I inhibit the binding of fibrinogen to blood platelets and inhibit aggregation of blood platelets. These compounds are useful as pharmaceutical agents for mammals, especially for humans. The compounds of this invention may be administered to patients where prevention of thrombosis by inhibiting binding of fibrinogen to the platelet membrane glycoprotein complex IIb/IIIa receptor is desired. They are useful in surgery on peripheral arteries (arterial grafts, carotid endarterectomy) and in cardiovascular surgery where manipulation of arteries and organs, and/or the interaction of platelets with artificial surfaces, leads to platelet aggregation and consumption. The aggregated platelets may form thrombi and thromboemboli. Compounds

of this invention may be administered to these surgical patients to prevent the formation of thrombi and thromboemboli.

Extracorporeal circulation is routinely used for cardiovascular surgery in order to oxygenate blood. Platelets adhere to surfaces of the extracorporeal circuit. Adhesion is dependent on the interaction between GPIIb/IIIa on the platelet membranes and fibrinogen adsorbed to the surface of the circuit. (Gluszko et al., Amer. J. Physiol., 1987, 252:H, pp 615-621). Platelets released from artificial surfaces show impaired hemostatic function. Compounds of this invention may be administered to prevent adhesion.

Other applications of these compounds include prevention of platelet thrombosis, thromboembolism, reocclusion and restenosis during and after thrombolytic therapy and prevention of platelet thrombosis, thromboembolism, reocclusion and restenosis after angioplasty of coronary and other arteries and after coronary artery bypass procedures. Compounds of this invention may also be used to prevent or modulate the progress of myocardial infarction, unstable angina and thrombotic stroke.

The compounds of Formula I may be administered to mammals, preferably humans, either alone or, preferably, in combination with pharmaceutically-acceptable carriers or diluents, optionally with known adjuvants, such as alum, in a pharmaceutical composition, according to standard pharmaceutical practice. The compounds can be administered orally or parenterally, including intravenous, intramuscular, intraperitoneal, subsutaneous and topical administration.

For oral use of a fibrinogen receptor antagonist according to this invention, the selected compounds may be administered, for example, in the form of tablets or capsules, or as an aqueous solution or suspension. In the case of tablets for oral use, carriers which are commonly used include lactose and corn starch, and lubricating agents, such as magnesium stearate, are commonly added. For oral administration in capsule form, useful diluents include lactose and dried corn starch. When aqueous suspensions are required for oral use, the active ingredient is combined with emulsifying and suspending agents. If desired, certain sweetening and/or flavoring agents may be added. For intramuscular, intraperitoneal, subcutaneous and intravenous use, sterile solutions of the active ingredient are usually prepared, and the pH of the solutions should be suitably adjusted and buffered. For intravenous use, the total concentration of solutes should be controlled in order to render the preparation isotonic.

The present invention also encompasses a pharmaceutical composition useful in the treatment of diseases related to platelet aggregation and fibrin formation, comprising the administration of a therapeutically effective but non-toxic amount of the compounds of Formula I, with or without pharmaceutically acceptable carriers or diluents.

Compositions of this invention include fibrinogen receptor antagonist compounds and pharmacologically acceptable carriers, e.g. saline, at a pH level e.g. 7.4, suitable for achieving inhibition of platelet aggregation. They may be combined with thrombolytic agents such as plasminogen activators or streptokinase in order to inhibit platelet aggregation. They may also be combined with anticoagulants such as heparin, aspirin or warfarin. Intravenous administration is presently contemplated as the preferred administration route. They are soluble in an aqueous medium, and may therefore be effectively administered in solution.

When a compound according to Formula I is used as a fibrinogen receptor antagonist in a human subject, the daily dosage will normally be determined by the prescribing physician with the dosage generally varying according to the age, weight, and response of the individual patient, as well as the severity of the patient's symptoms.

In one exemplary application, a suitable amount of compound is intravenously administered to a heart attack victim undergoing angioplasty. Administration occurs during or several minutes prior to angioplasty, and is in an amount sufficient to inhibit platelet aggregation, e.g. an amount which achieves a steady state plasma concentration of between about 0.01-30 $\mu$M per kilo, preferably between about 0.3-3 $\mu$M per kilo. When this amount is achieved, an infusion of between about 1-100 $\eta$M per kilo per min., preferably between about 10-30 $\eta$M per kilo per min. is maintained to inhibit platelet aggregation. Should the patient need to undergo bypass surgery, administration may be stopped immediately and will not cause complications during surgery that would be caused by other materials such as aspirin or monoclonal antibodies, the effects of which last hours after cessation of administration.

The present invention also includes a pharmaceutical composition comprising compounds of the present invention and tissue type plasminogen activator or streptokinase. The invention also includes a method for promoting thrombolysis and preventing reocclusion in a patient which comprises administering to the patient an effective amount of compositions of the invention.

The compounds of the Formula I are prepared according to the reaction Schemes set forth below:

## SCHEME I

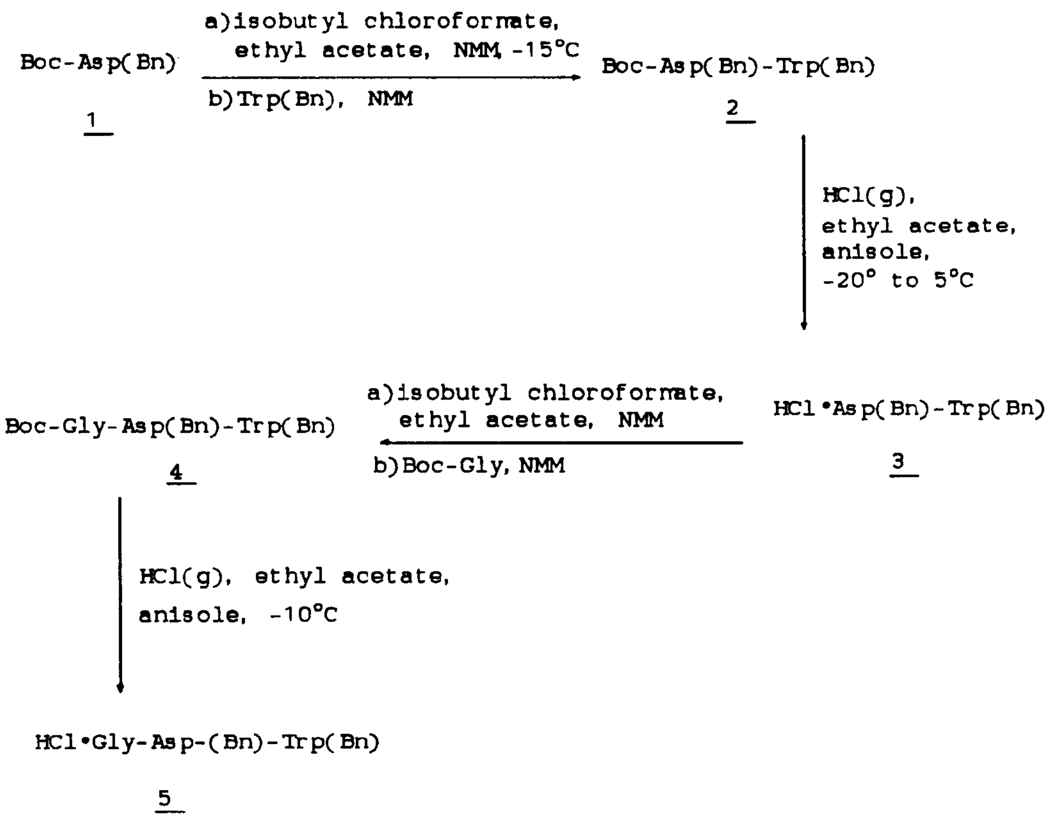


Bn=benzyl

NMM = N-methylmorpholine

## SCHEME II

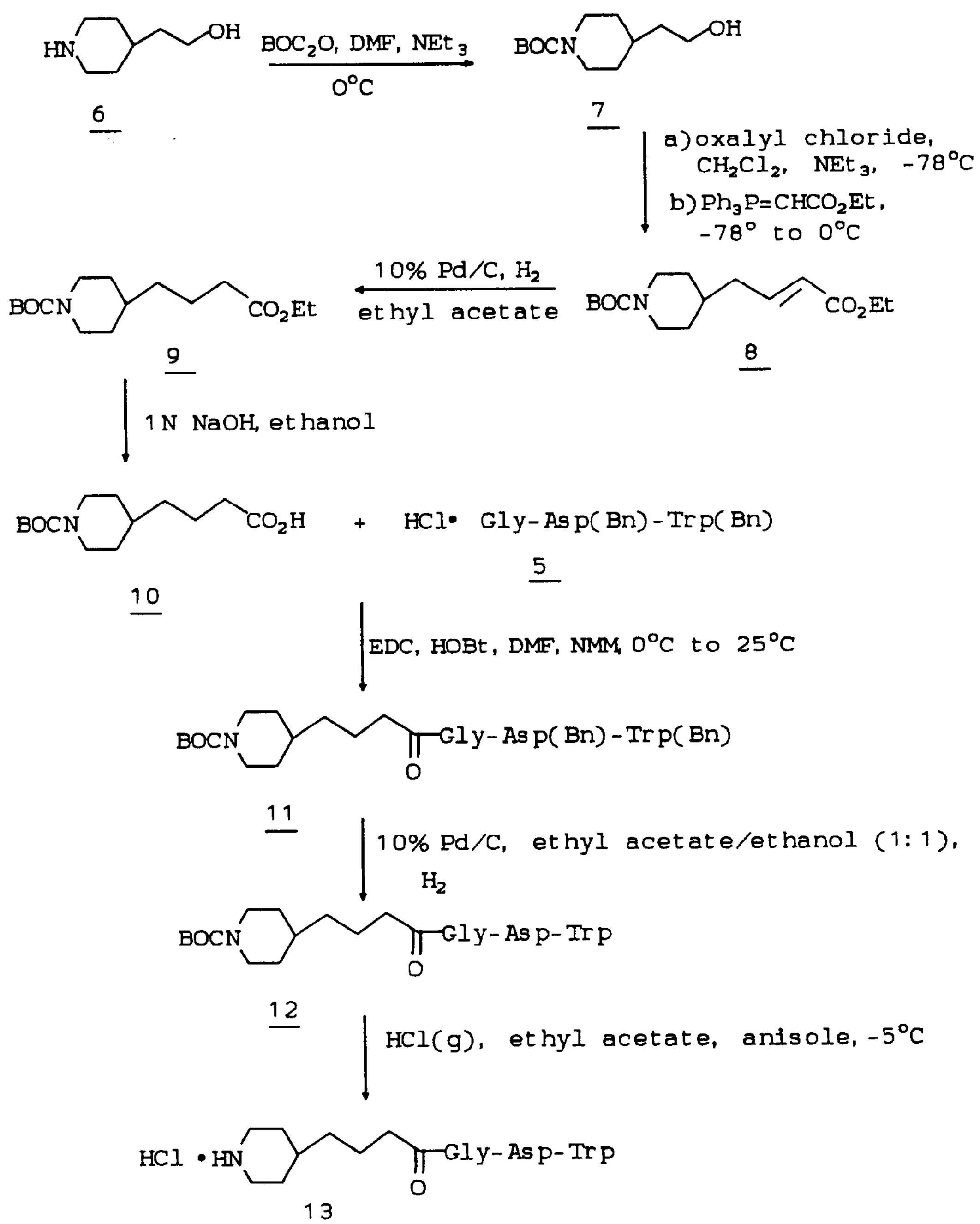


The present invention may be embodied in other specific forms without departing from the spirit or essential attributes thereof. Examples provided are intended to assist in a further understanding of the invention. Particular material employed, species and conditions are intended to be further illustrative of the invention and not limitative of the reasonable scope thereof. All flash column chromatography and TLC analysis were performed on silica gel. All $^1H$ NMR spectra were obtained on a Varian 300 MHz instrument.

EXAMPLE 1

Preparation of Boc-Asp(Bn)-Trp(Bn) 2

To a stirred solution of Trp(Bn) 1 (25 g, 80 mmol), N-methylmorpholine (NMM; 9 mL, 85 mmol), and ethyl acetate (800 mL) at -15°C (ice/ethanol) was added isobutyl chloroformate (10.4 mL, 80 mmol) in a stream. After 20 minutes Trp(Bn) (25 g, 75 mmol) was added to the heterogenous mixture followd by NMM (9.3 mL). This was diluted with $H_2O$ after 2.0 hours and transfered to a separatory funnel. After shaking and seperation, the organic portion was washed sequentially with 5% $KHSO_4$, sat. $NaHCO_3$ and brine, dried ($MgSO_4$), and concentrated to give the dipeptide 2 (41 g) as a brown oil.
TLC Rf=0.73 (50% ethyl acetate/hexanes).

EXAMPLE 2

Preparation of Asp(Bn)-Trp(Bn)·HCl 3

Hydrogen chloride gas was vigorously bubbled through an ethyl acetate (670 mL) solution of 2 (40 g, 67 mmol) and anisole (14.5 mL, 135 mmol) which was mechanically stirred. The internal temperature was initially -40°C (isopropanol/dry ice) but rose to -20°C over a 30 minute period. The HCl addition was curtailed and the reaction was allowed to warm to -5°C over a 1.0 hour period. Nitrogen was then bubbled through the reaction for 2.5 hours to remove excess HCl. Ether (400 ml) was added to the heterogeneous mixture to effect efficient precipitation. The solid was collected by filtration, washed with ether, then after storing in vacuo overnight gave the HCl salt 3 (34.8 g) as an off-white solid.
TLC Rf=0.51 (9:1:1 $CH_2Cl_2/CH_3OH/AcOH$).

EXAMPLE 3

Preparation of Boc-Gly-Asp(Bn)-Trp(Bn) 4

To a stirred solution of ethylacetate (650 mL) Boc-Gly (12.3 g, 70 mmol), and NMM (8.2 mL, 75 mmol) at -15°C (ethanol/ice) was added isobutyl chloroformate (9.1 mL, 70 mmol) in a stream to effect a fine precipitate. After 15 minutes amine·HCl 3 (34 g, 64 mmol) in DMF (100 mL) was added followed by NMM (8.2 mL). After 1.5 hours the reaction was washed with $H_2O$, 5% $KHSO_4$, sat. $NaHCO_3$, and brine, dried ($MgSO_4$), and concentrated. The resulting oil was triturated into a white solid with ether. Filtration gave the tripeptide 4 (37.8 g) as a white solid. TLC Rf=0.27 (50% ethyl acetate/hexanes);
$^1H$ NMR ($CDCl_3$) δ 8.76 (d, J = 7Hz, 1H), 8.66 (d, J = 7Hz, 1H), 8.10 (m, 2H), 7.49 (d, J = 8Hz, 1H), 7.18-7.40 (m), 7.08 (t, J = 7Hz, 1H), 7.00 (t, J = 7Hz, 1H) 5.07 (d, J = 2Hz, 2H), 5.02 (d, J = 3Hz, 2H), 4.80 (m, 1H), 4.56 (m, 1H), 3.70 (m, 2H), 3.50 (m, 2H), 3.14 (m, 2H), 2.73 (dd, J = 17 and 4 Hz, 1H), 2.56 (dd, J = 17 and 8Hz, 1H), 2.50 (m, 2H).

EXAMPLE 4

Preparation of Gly-Asp(Bn)-Trp(Bn)·HCl (5)

Into a mechanically stirred mixture of the tripeptide 4 (37 g, 56 mmol), ethylacetate (500 mL) and anisole (12.1 mL, 0.11 mol) at -30°C was bubbled HCl gas for 15 minutes. The cooling bath was removed and argon was bubbled through the yellow solution for 2.0 hours. The resulting off-white solid was collected by filtration and washed with ethyl acetate, then dried in vacuo for 5.0 hours to give the amine·HCl 5 (30 g).
TLC Rf=0.42 (9:1:1 $CH_2Cl_2/CH_3OH/AcOH$).

EXAMPLE 5

Preparation of N-Boc-4-piperidineethanol 7

To a stirred solution of 4-piperidineethanol 6 (18.7 g, 0.14 mol) and DMF (200 mL) at 0°C was added N-tert-butoxycarbonylanhydride (31 g, 0.14 mol). After 1.0 hour the cooling bath was removed and the reaction mixture stirred for 20 hours. The reaction mixture was diluted with ether and then washed with $H_2O$ (2x) and brine, dried ($MgSO_4$), and concentrated to furnish 7 (26 g) as a colorless oil.

TLC RF=0.25 (40% ethyl acetate/hexanes);
$^1$H NMR ($CDCl_3$) δ 4.09 (bs, 2H), 3.72 (t, J = 7Hz, 2H), 2.70 (m, 2H), 1.75-1.10 (m, 7H), 1.46 (s, 9H).

EXAMPLE 6

Preparation of Ethyl 4-(N-Boc-4-piperidyl)transcrotonate 8

To a stirred solution of oxalyl chloride (0.43 mL, 5.0 mmol) in $CH_2Cl_2$ (20 mL) at -78°C was added DMSO (0.52 ml, 7 mmol) dropwise. After gas evolution subsided (5 minutes) the alcohol 7 (0.8 g, 3.5 mmol) in $CH_2Cl_2$ (20 mL) was added in a stream. After 20 minutes triethylamine (1.7 mL, 12 mmol) was added dropwise and then the cooling bath was removed. After 20 minutes (carbethoxymethylene)triphenylphosphorane (1.4 g, 4.0 mmol) was added. After 2.0 hours the reaction mixture was diluted with petroleum ether and then washed sequentially with $H_2O$, 5% $KHSO_4$, and brine, dried ($MgSO_4$), and concentrated. Flash chromatography (silica, 15% ethyl acetate/hexanes) gave the ester 8 (0.57 g) as a colorless oil.
TLC Rf=0.79 (50% ethyl acetate/hexanes);
$^1$H NMR ($CDCl_3$) δ 6.91 (dt, J = 16 and 7Hz, 1H), 5.81 (bd, J = 7Hz, 1H), 4.18 (g, J = 7Hz, 2H), 4.08 (m, 2H), 2.67 (m, 2H), 2.14 (t, J = 7Hz, 2H), 1.70-1.05 (m, 5H), 1.44 (s, 9H), 1.28 (t, J = 7H, 3H).

EXAMPLE 7

Preparation of Ethyl 4-(N-Boc-4-piperidyl) butyrate 8

The olefin 8 (26 g, 87 mmol) in ethyl acetate (500 mL) was hydrogenated, at ambient temperature, under a hydrogen atmosphere (1 atm) in the presence of 10% Pd/C (5.0 g) overnight. The reaction mixture was then purged with argon followed by filtration through a celite pad. Concentration of the filtrate followed by flash chromatography (silica, 10% ethyl acetate/hexanes) gave the ester 9 (24 g) as a crystalline solid.
TLC Rf=0.52 (20% ethyl acetate/hexanes);
$^1$H NMR ($CDCl_3$) δ 4.16 (q, J = 7Hz, 2H), 4.10 (m, 2H), 2.69 (m, 2H), 2.31 (t, J = 7Hz, 2H), 1.68 (m, 4H), 1.38 (s, 9H), 1.40 (m, 1H), 1.11 (m, 2H).

EXAMPLE 8

Preparation of 4-(N-Boc-4-piperidyl)butanoic acid 10

A solution of ester 9 (19 g, 63 mmol), ethanol (300 mL) and IN NaOH (100 mL, 100 mmol) was stirred at ambient temperature for 2.5 hours followed by concentration. The residue was diluted with 5% $KHSO_4$ and ethyl acetate then transfered to a separatory funnel. The phases were shaken then separated and the organic portion was washed with brine, dried ($MgSO_4$), and concentrated to give the acid 10 (18 g) as a colorless oil that crystallized upon standing.
TLC Rf=0.68 (ethyl acetate).

EXAMPLE 9

Preparation of N-Boc-Pib-Gly-Asp(Bn)-Trp(Bn) 11

A stirred solution of acid 10 (3.5 g, 12.9 mmol), tripeptide (5) (7.1 g, 11.7 mmol), 1-hydroxybenzotriazole hydrate (2.1 g, 15.5 mmol), N-methylmorpholine (4.9 mL, 44 mmol), and DMF (130 mL) at 0°C was treated with EDC (3.2 g, 16.7 mmol) followed by removal of the cooling bath. After 18 hours the yellow solution was diluted with ethyl acetate and then washed sequentially with $H_2O$, 5% $KHSO_4$, sat. $NaHCO_3$, and brine, dried ($MgSO_4$), and concentrated. Flash chromatography (silica, 4 to 4.5% isopropanol/ $CHCl_3$) afforded 11 (6.3 g) as a foam.
TLC Rf=0.24 (5% isopropanol/$CHCl_3$);
$^1$H NMR ($CDCl_3$) δ 8.54 (bs, 1H), 7.50 (d, J = 8Hz, 1H), 7.35-6.95 (m, 16H), 6.81 (d, J = 2Hz, 1H), 5.98 (t, J = 5Hz, 1H) 5.06 (m, 4H), 4.86 (m, 1H), 4.80 (m, 1H), 4.05 (m, 2H), 3.62 (m, 2H), 3.28 (m, 2H), 2.89 (dd, J = 17 and 5Hz, 1H), 2.63 (m, 3H), 2.13 (t, J = 7Hz, 2H), 1.68-0.98 (m, 7H), 1.44 (s, 9H).

EXAMPLE 10

Preparation of N-Boc-Pib-Gly-Asp-Trp 12

A mixture of the dibenzylester 11 (6.1 g, 7.4 mmol), ethanol (200 mL), ethyl acetate (200 mL), and 10% Pd/C (1.2 g) was stirred at ambient temperature, under a hydrogen atmosphere (1 atm) for 3 hours. The reaction was purged with argon, then filtered through a celite pad to give a pink oil after concentration. Flash chromatography (silica, 10:0.8:0.8 $CHCl_3/AcOH/CH_3OH$) furnished the diacid 12 (3.8 g) as a slightly pink oil. TLC Rf=0.30 (9:1:1 $CH_2Cl_2/AcOH/CH_3OH$).

EXAMPLE 11

Preparation of Pib-Gly-Asp-Trp·HCl 13

To a suspension of the diacid 12 (3.7 g, 5.8 mmol) in ethyl acetate (150 mL) at -60°C was vigorously bubbled HCl gas for 10 minutes until the internal temperature reached -20°C. The initial suspension went into solution as a beige precipitate began to appear. The reaction mixture was then allowed to warm from -20°C to -5°C over a 20 minute period. Argon was then passed through the suspension for 30 minutes and the resulting solid collected by filtration washing with ethyl acetate. After storing the resulting solid overnight under vacuum, the amine·HCl 13 (3.0 g) was obtained as a beige solid.
TLC Rf=0.42 (9:1:1 $CH_3OH/NH_4OH/H_2O$);
$^1H$ NMR ($CD_3Cl$) δ 7.82 (d, J=8Hz, 1H), 7.58 (d, J=8Hz, 1H), 7.37 (s, 1H), 7.32 (t, J=8Hz, 1H), 7.26 (t, J=8Hz, 1H), 5.00 (t, J=6Hz, 1H), 4.90 (t, J=6Hz, 1H), 4.03 (s, 2H), 3.53 (m), 3.10 (m, 2H), 2.98 (m, 2H), 2.50 (t, J=7Hz, 2H), 2.12 (m, 2H), 1.87 (m, 2H), 1.78 (m, 1H), 1.57 (m, 2H).

## Claims

1. A fibrinogen receptor antagonist of the Formula:

$$(X-(CH_2)_m-Y-(CH_2)_n-\overset{O}{\overset{\|}{C}})-Gly-Asp-AA \qquad I$$

wherein:
X is

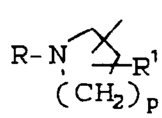 , 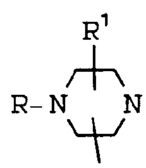 ,

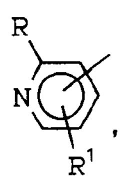 , 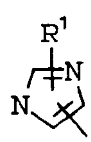 ,

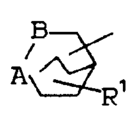 or 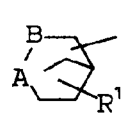 ;

A or B is N-R, provided that when A is N-R, B is -CH2-, and that when B is N-R, A is

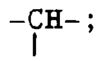

Y is

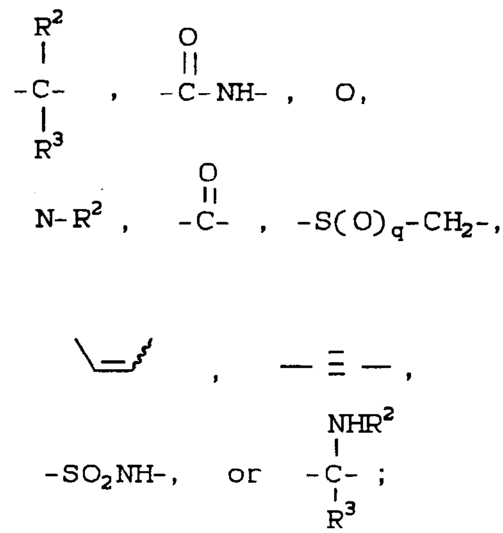

R, $R^1$, $R^2$ and $R^3$ are independently alkyl, aryl, alkylaryl, substituted alkyl, or H;

AA is an L-Amino Acid;
m is 1-10;
n is 0-9;
q is 0-2;
p is 1-6;

and the pharmaceutically acceptable salts thereof.

**2.** A fibrinogen receptor antagonist of Formula I in Claim 1, wherein:

X is

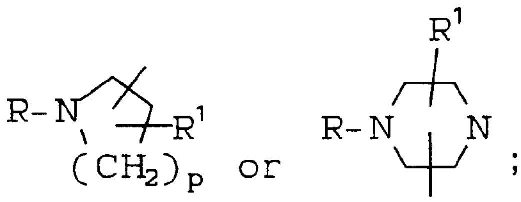

Y is

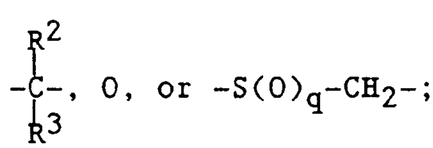

R, $R^1$, $R^2$ and $R^3$ are independently alkyl, aryl, alkylaryl, substituted alkyl, or H;

AA is an L-Amino Acid;
m is 1-5;
n is 0-5;
q is 0-2;
p is 2-4;

and the pharmaceutically acceptable salts thereof.

3. The fibrinogen receptor antagonist compound of Claim 1 which is

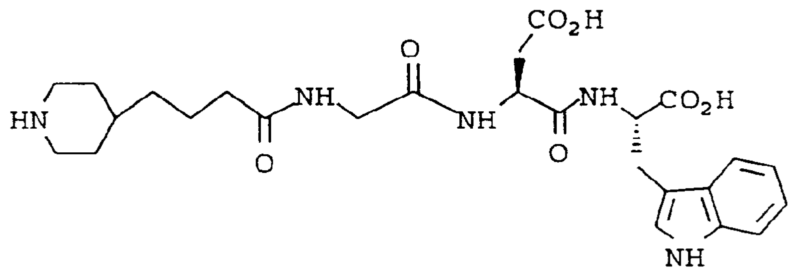

4. A pharmaceutical composition comprising a compound of Claim 1 and a pharmaceutically acceptable carrier.

5. The use of a compound as claimed in claim 1 for the preparation of a medicament for inhibiting the binding of fibrinogen to blood platelets.

6. The use of a compound as claimed in claim 1 for the preparation of a medicament for inhibiting aggregation of blood platelets.

7. A pharmaceutical composition comprising a compound of claim 1 in combination with a thrombolytic agent and a pharmaceutically acceptable carrier.

8. The composition of claim 7, wherein the thrombolytic agent is a plasminogen activator or streptokinase.

9. A pharmaceutical composition comprising a compound of claim 1 in combination with an anticoagulant and pharmaceutically acceptable carrier.

10. The composition of claim 9, wherein the anticoagulant is heparin, aspirin or warfarin.